# EUROPEAN PATENT SPECIFICATION

(11) **EP 2 046 390 B1**
(45) Date of publication and mention of the grant of the patent: **10.04.2013**
(21) Application number: 07766368.0
(22) Date of filing: 27.07.2007
(51) Int. Cl.: A61K 47/32

(54) **LINSEED EXTRACT MEDICAMENT FOR APPLICATION TO THE EYE**
LEINSAMENEXTRAKT-MEDIKAMENT ZUR APPLIKATION AUF DAS AUGE
MÉDICAMENT À BASE D'EXTRAIT DE GRAINES DE LIN POUR APPLICATION OCULAIRE

(30) Priority: 28.07.2006 GB 0615064
(43) Date of publication of application: 15.04.2009
(73) Proprietor: Sinclair Pharmaceuticals Limited, London W1T 2RQ (GB)
(72) Inventor: MASTRONDONATO, Marco, I-20124 Milano (IT)
(74) Representative: Jappy, John William Graham
(86) International application number: PCT/GB2007/002851
(87) International publication number: WO 2008/012548

(56) References cited:
- EP-A1- 0 511 181
- WO-A-2005/074957
- US-A1- 2003 143 288
- LUDWIG ET AL: "The use of mucoadhesive polymers in ocular drug delivery" ADVANCED DRUG DELIVERY REVIEWS, AMSTERDAM, NL, vol. 57, no. 11, 3 November 2005 (2005-11-03), pages 1595-1639, XP005114833 ISSN: 0169-409X

## Description

### Field of the Invention

This invention relates to methods and products for application to the eye, in particular for treating Dry Eye Syndrome.

### Background to the Invention

Dry Eye, also known as Dry Eye Syndrome, is the common name given to Keratoconjunctivitis sicca, which is also referred to as Keratitis sicca and Xerophthalmia. The condition is caused by either a lack of tears, or an incorrect tear composition, so that the eyes are insufficiently lubricated and become uncomfortable.

The tear film consists of three layers. The outer, oily layer of the tear film is produced by the meibomian glands in the eyelids and reduces evaporation of the tears. The thick, middle, watery layer is made by the lacrimal gland above the upper eyelid and washes away irritants. The inner, mucus layer is secreted by the goblet cells in the conjunctiva of the eyelids and helps the tear film stick to the cornea.

In Dry Eye, eyes can feel persistently gritty, itchy, burning, and painful. These sensations often worsen as the day goes on. Stringy mucus may appear in or around the eyes, while Patients with the most severe disease are at increased risk of developing corneal infection, scarring or ulceration.

Dry Eye's prevalence increases with age, so that it is extremely common in older people of both sexes. The condition is more prevalent in women than men. About six million women and three million men in the U.S. have moderate or severe symptoms of the disease.

Dry Eye is an important public health problem. One reason for this is that it is very common; visits for Dry Eye Syndrome are one of the leading reasons for patients to seek eye care. This is because its symptoms are very bothersome and lead to a decreased quality of life, reduced work capacity and poorer psychological health. Furthermore, Dry Eye Syndrome is associated with a decreased ability to perform activities that require visual attention, such as reading and driving a car.

Many practitioners feel unable to help sufferers of Dry Eye, as they do not believe that they can confidently recommend an artificial tear product that is effective in the maintenance of the tear film and/or at relieving symptoms.

Drugs can be delivered topically to the eye and treatment of eye conditions can therefore be achieved by applying a drug or treatment directly to the eye. However, due to the delicate nature of the eye and surrounding tissues, effective vehicles for delivering drugs to the eye have been problematic to identify.

US2003/0143288 discloses anti-inflammatory compositions that include effective amounts of licorice and linseed extracts for use in the treatment of xerostomia.

There is clearly a need for an effective treatment for Dry Eye Syndrome. The treatment should ideally provide immediate and long-lasting relief, be nontoxic, be non-blurring to vision and be simple to apply. There is also a need for effective compositions for delivering drugs to the eye.

### Summary of the Invention

The present invention is based on the surprising realisation that linseed extract is an effective treatment for Dry Eye, and can also be used to deliver drugs to the eye.

According to a first aspect of the invention, linseed extract is used in the manufacture of a medicament to treat Dry Eye.

According to a second aspect of the invention, a composition comprising linseed extract is used in the treatment of dry eye.

### Detailed Description of the Invention

The present invention involves the application of linseed extract to the eye. As used herein, the term "eye" is to be given its usual meaning in the art, i.e. the organ of sight.

Linseed, also known as flax or flaxseed, is the seed of a plant of the genus linum in the family linaceae. The term "linseed" includes the seeds of approximately 150 species of plant, all of which are within the scope of the invention. A common species is Linum usitatissimum. All varieties of all species are within the scope of the invention. A preferred variety is the "Tadorna" variety of Linum usitatissimum. The Tadoma variety is known in the art and the preparation of Tadoma linseed extract is described in Example 5 of US Patent No. 5,260,282. Linseed extract is well known in the art. As used herein, the term "linseed extract" is to be given its usual meaning, i.e. the product of buffer extraction of linseed.

The linseed extract used in the invention is obtainable by simple extraction in water of polysaccharides and proteins directly from linseed as described in US Patent No. 5,260,282, in particular Examples 1 to 5 of US 5,260,282. Any extraction method may be employed, for example extraction using organic solvent, water, a supercritical fluid or a mixture thereof. When a mixture of an organic solvent and water is used, preferably a protic solvent such as ethanol is used. Methods of preparing linseed extract, in particular dried linseed extract, are disclosed by WO- A-2005/074957.

In a preferred embodiment, the linseed extract is dried, more preferably the extract is solid. Methods of producing dried linseed extract are known in the art, for example as disclosed in WO-A-2005/074957. Preferred methods of drying a linseed extract are spray drying and freeze drying. As used herein, the term "dry" means that the extract is substantially free of water. Preferably, the dried extract has less than 10% water by weight, more preferably less than 5% water by weight, more preferably less than 2% water by weight, e.g. less than 1% water by weight. The process of drying the linseed extract is further thought to have a surprising effect on the physical properties of the extract, providing increased adsorption to tissue, in particular mucosal tissue. This provides a medicament with improved resident time in the eye and, additionally, has improved film-forming properties.

Preferably, the linseed extract is water-soluble or water-dispersible and has an adsorption of at least 1.2mg/m² wherein the adsorption is measured by contacting an aqueous solution or dispersion of the extract (i.e. a composition of the invention) with a silica substrate, rinsing the silica substrate and then measuring the adsorption by ellipsometry. Preferably, the extract or composition according to the Invention has an adsorption in the range 1.3 to 5mg/m², more preferably 1.4 to 4mg/m², yet more preferably 1.5 to 3mg/m². When the extract has been prepared by spray drying, improved adsorption characteristics are obtained and the adsorption is preferably from 1.75 to 2.5mg/m², more preferably about 2mg/m². In the adsorption measurement method, there is a contact time between when the aqueous solution or dispersion of the extract contacts the silica substrate and when the silica substrate is rinsed. This contact time is preferably from 100 to 3000 seconds, more preferably 500 to 2500 seconds, yet more preferably 1000 to 2000 seconds.

A first aspect of the invention involves the use of linseed extract on the treatment of Dry Eye. A composition comprising linseed extract is an effective "Artificial Tear" composition. It should be noted that, although dried linseed extract is useful as an ingredient when preparing the medicament or composition, the medicament itself, that contacts the eye, is not dry and contains a substantial amount of water or other pharmaceutically acceptable buffer. Preferably, the composition that is applied to the eye is an aqueous solution or gel.

As used herein, the term "Dry Eye" refers to Keratoconjunctivitis sicca, Keratitis sicca and Xerophthalmia. The condition is characterised by either a lack of tears, or an incorrect tear composition, so that the eyes are insufficiently lubricated and become uncomfortable. Dry Eye is increasingly caused by air conditioning, computer use and air pollution such as smog.

Without wishing to be bound by theory, the inventor believes that linseed extract is advantageous as it contains mucins and mucin-like proteins, which provide lubrication, and has the ability to hold significant amounts of water, which keeps the eye hydrated for a sustained period.

Dry Eye may be treated by contacting the eye directly with a linseed extract. The eye is contacted with a composition, preferably an aqueous composition, comprising linseed extract and optionally other components. A preferred additional component is polyvinylpyrrolidone (CAS No. 9003-39-8).

A further preferred ingredient is at least one electrolyte that causes the composition to have the same electrolyte balance as "normal" human tears, or which is hypertonic relative to normal human tears. Preferably, a composition of the invention has an osmolarity of 400 mOsm/litre or less, preferably 300mOsm/litre or less such as 270-300 mOsm/litre, more preferably less than 200 mOsm/litre, e.g. between 100 and 150 mOsm/litre. Formulations for application to the eye that are hypertonic are known in the art, for example

TheraTears™, available from Advanced Vision Research, Inc., 660 Main Street, Woburn, MA 01801 , USA.

In a further preferred embodiment, hyaluronic acid or a pharmaceutically acceptable salt thereof is included in a composition used in the invention. As used herein, the term "hyaluronic acid" is to be given its usual meaning in the art. Alternative names for hyaluronic acid include hyaluronan and hyaluronate. For the avoidance of doubt, hyaluronic acid is a polymer of disaccharides, each disaccharide consisting of D-glucuronic and D-n-acetyl glucosamine, linked via alternating β-1, 4 and β-1, 3 glycosidic bonds. Hyaluronic acid can be many thousand disaccharide repeats in length, with polymers ranging from 5 thousand to 20 million Da in vivo. According to the present invention, any sized hyaluronic acid may be used; the term "hyaluronic acid" includes both high molecular weight hyaluronic acid and low molecular weight hyaluronic acid. These terms are well known in the art; for the avoidance of doubt, low molecular weight hyaluronic acid has a molecular weight of less than 400 kDa, more preferably less than 100 kDa, yet more preferably less than 20 kDa, for example between 1 and 10 kDa. High molecular weight hyaluronic acid has a molecular weight greater than 400 kDa, more preferably greater than 800 kDa.

Hyaluronic acid or a salt of hyaluronic acid can be used according to the present invention. Preferably, the salt is a pharmaceutically acceptable salt. Examples of pharmaceutically acceptable salts are alkali metal salts such as sodium or potassium salt or alkaline earth metal salts such as magnesium or calcium salt.

Preferred compositions used in the present invention include a composition comprising linseed extract and hyaluronic acid, a composition comprising linseed extract and polyvinylpyrrolidone, and a composition comprising linseed extract, hyaluronic acid and polyvinylpyrrolidone. Each of the ingredients can be present in the composition in an amount that will be apparent to the skilled person. A preferred amount of polyvinylpyrrolidone is 0.01% w/v to 10% w/v, preferably 0.5% w/v to 5% w/v, more preferably 0.5% w/v to 2% w/v, such as 1 % w/v. A preferred amount of linseed extract is 0.01% w/v to 10% w/v, preferably 0.5% w/v to 5% w/v, more preferably 0.5% w/v to 1% w/v. A preferred amount of hyaluronic acid is 0.01% w/v to 5% w/v, preferably 0.1% w/v. Additional components that can be included are indicated in Tables 1 to 3.

At least one omega-3 fatty acid may also be included in a composition of the invention. Omega-3 fatty acids are well-known in the art as a family of polyunsaturated fatty acids characterised by a carbon-carbon double bond in the omega-3 position. Examples of omega-3 fatty acids include linolenic acid, eicosapentanoic acid and docosahexaenoic acid.

A pharmaceutical composition comprising a linseed extract according to the invention and one or more pharmaceutically acceptable excipients is within the scope of the invention. Examples of suitable excipients include diluents, glidants, preservatives, gums, coatings, binders, disintegrants, lubricants, suspending agents, solvents, dispersants, colourants, anti-adherents, surfactants, plasticisers, emulsifiers, chelators and emollients. Preferably the composition contains no artificial preservatives. In more severe cases of Dry Eye, involving inflammation of the cornea, it is preferable to include in a composition according to the invention a topical anti-inflammatory agent. This can be a steroidal or non-steroidal anti-inflammatory agent (NSAID) such as aspirin or ibuprofen (i.e. cycloxygenase inhibitors).

The composition of the invention can be prepared in any suitable formulation for topical application to the eye. Suitable formulations include pharmaceutically-acceptable liquids and gels, creams and ointments. The composition can be hydrophilic or hydrophobic. The composition can be an aqueous composition, although other suitable solvents, such as alcohols or other organic solvents, may be used. A combination of solvents may also be used. Preferably, the composition is sterile. Formulations that change phase when applied to the eye are also suitable; for example, a gel that transforms into a liquid (due to the shearing effect of blinking), and a liquid that transforms into a gel on contact with the eye are known in the art. The skilled person will recognise that liquids and gels are preferred formulations for application to the eye.

The composition of the invention is preferably packaged in a container, preferably a vial or bottle, that contains a dropper to allow application of single droplets to the eye. Preferably, the dropper is an integral part of the container or the container lid. The dropper preferably dispenses drops having an average volume between 10 µl and 500 µl, more preferably approximately 40 µl.

Tables 1 to 3 show preferred compositions that can be used to treat Dry Eye.

**Table 1**

| | |
|---|---|
| POLYVINYLPYRROLIDONE | 0,5 % w/v |
| SODIUM CHLORIDE | 0,65 % w/v |
| LINSEED DRIED EXTRACT | 0,5 % w/v |
| SODIUM PHOSPHATE | 0,034 % w/v |
| SODIUM DIPHOSPHATE ANHYDROUS | 0,236 % w/v |
| DISTILLED WATER | Up to 100 % |

**Table 2**

| | |
|---|---|
| POLYVINYLPYRROLIDONE | 0,5 % w/v |
| SODIUM CHLORIDE | 0,54 % w/v |
| LINSEED DRIED EXTRACT | 1 % w/v |
| SODIUM PHOSPHATE | 0,066 % w/v |
| SODIUM DIPHOSPHATE ANHYDROUS | 0,471 % w/v |
| DISTILLED WATER | Up to 100 % |

**Table 3**

| | |
|---|---|
| POLYVINYLPYRROLIDONE | 2 % w/v |
| SODIUM CHLORIDE | 0,54 % w/v |
| LINSEED DRIED EXTRACT | 1 % w/v |
| SODIUM PHOSPHATE | 0,066 % w/v |
| SODIUM DIPHOSPHATE ANHYDROUS | 0,4715 % w/v |
| DISTILLED WATER | Up to 100 % |

A method for treating Dry Eye comprises contacting the eye of a patient suffering from Dry Eye with a composition according to the invention, thereby relieving the symptoms of Dry Eye in the patient. The eye can remain in contact with a contact lens while the composition is applied, i.e. if the subject is wearing contact lenses, there is no need to remove these to apply a composition of the invention.

A composition according to the invention can be applied to the eye once daily. The compositions of the invention can advantageously provide relief from Dry Eye for a number of hours, for example 6 hours or more in some patients, and a once daily application will improve patient compliance. In an alternative embodiment, a composition according to the invention is applied to the eye more often than once daily, for example two, three or more times daily, such as hourly or every 2-3 hours. This multiple application has the advantage that regular application of a composition according to the invention washes away toxins and/or inflammatory agents which can result from Dry Eye Syndrome.

A second aspect of the invention relates to the use of a composition as described above, in delivering at least one drug to the eye. A composition of the invention is useful for delivering drugs across the cornea, sclera and conjunctiva.

In a preferred embodiment, drugs intended for sustained release are delivered to the eye. Drugs that are intended for delivery to the eye may therefore be incorporated into a composition of the invention.

Preferably, the drug that is delivered to the eye is for treatment of an eye condition or disease; in this embodiment, the composition is for topical treatment of the eye. More preferably, the drug is a topical antimicrobial, preferably antibacterial, used to treat an eye infection or a drug that is used to treat Dry Eye, such as Topical Cyclosporine A. However, the present invention can also be used to deliver a drug to the eye that does not exert its effect on the eye, i.e. a composition of the invention can be used to deliver systemic drugs via the eye.

Any drug may be incorporated, examples of suitable drugs include anti-inflammatory drugs (e.g. ibuprofen, indomethacin, naproxen, diclofenac, tolfenamic acid, piroxicam), analgesics (e.g. buprenorphine, codeine, fentanyl, morphine, hydromorphone), tranquilizers (e.g. diazepam, droperidol, fluspirilene, haloperidol, lorazepam, propiomazin), cardiac glycosides (e.g. digoxin, ouabain), narcotic antagonists (e.g. naloxone, nalorphine), antiparkinsonism agents (e.g. bromocriptine, biperiden, benzhexol, benztropine), antidepressants (e.g. imipramine, nortriptyline, protriptylene), antineoplastic agents and immunosuppressants (e.g. bleomycin, cyclosporin A, fluorouracil, mercaptopurine, methotrexate, mitomycin), antiviral agents (e.g. idoxuridine, acyclovir, interferons, vidarabin), antibiotic agents (e.g. clindamycin, erythromycin, fusidic acid, gentamicin), appetite suppressants (e.g. fenfluramine, mazindol, phentermin), antiemetics (e.g. metoclopramide, droperidol, haloperidol, promethazine), antihistamines (e.g. chlorpheniramine, terfenadine, triprolidine), antimigrane agents (e.g. dihydroergotamine, ergotamine, pizotyline), coronary, cerebral or peripheral vasodilators (e.g. nifedipine, diltiazem), antianginals (e.g. glyceryl trinitrate, isosorbide dinitrate, molsidomine, verapamil), calcium channel blockers (e.g. verapamil, nifedipine, diltiazem, nicardipine), hormonal agents (e.g. calcitonin, estradiol, estron, estriol, polyestradiol, polyestriol, dienestrol, diethylstilbestrol, progesterone, dydrogesterone, cyproterone, danazol, testosterone, calcitonin), contraceptive agents (e.g. ethinyl estradiol, lynestrenol, etynodiol, norethisterone, mestranol, norgestrel, levonorgestrel, desogestrel, medroxyprogesterone), antithrombotic agents (e.g. fragmin, heparin, warfarin), diuretics (e.g. hydrochiorothiazide, flunarizine, minoxidil), antihypertensive agents (e.g. propanolol, metoprolol, cionidine, pindolol), chemical dependency drugs (e.g. nicotine, methadone), local anaesthetics (e.g. lidocaine, prilocaine, benzocaine), corticosteroids (e.g. beclomethasone, betamethasone, clobetasol, desonide, desoxymethasone, dexamethasone, diflucortolone, flumethasone, fluocinolone acetonide, fluocinonide, hydrocortisone, methyiprednisolon, triamcinolone acetonide, budesonide, halcinonide), dermatological agents (e.g. nitrofurantoin, dithranol, clioquinol, hydroxyquinoline, isotretinoin, methoxsalen, methotrexate, tretinoin, trioxsalen, salicylic acid, penicillamine), and the like.

Examples of specific active substances are steroids such as estradiol, progesterone, norethindrone, levonorgestrol, ethynodiol, levenorgestrel, norgestimate, gestanin, desogestrel, 3-keton-desogestrel, demegestone, promethoestrol, testosterone, spironolactone, and esters thereof; a nitro compound such as amyl nitrates, nitroglycerine and isosorbide nitrates; an amine compound such as prilocaine, oxybutyninchloride, lidocaine, benzocaine, nicotine, chlorpheniramine, terfenadine, triprolidine, propanolol and metoprolol; an oxicam derivative such as piroxicam; a mucopolysaccharide such as thiomucase an opioid such as morphine and morphine-like drugs such as buprenorphine, oxymorphone, hydromorphone, levorphanol, fentanyl and fentanyl derivatives and analogs; a prostaglandin such as a member of the PGA, PGB, PGE and PGF series, such as e.g. misoprostol and enaprostil; a benzamide such as metoclopramide and scopolamine; a peptide such as growth-hormone releasing factors, growth factors (EGF, TGF, PDGF and the like), somatostatin and insulin; a xanthine such as caffeine and theophylline; a catechloamine such as ephedrine, salbutamol and terbutaline; a dihydropyridine such as nifedipine; a thiazide such as hydrochlorothiazide and flunarizine; a sydnonimine such as molsidomine; and a sulfated polysaccharide such as heparin.

As indicated above, preferred drugs are those that are used to treat microbial infections of the eye. Preferably, the anti-microbial is in the form of a topical preparation for contact with the eye. The term "anti-microbial" includes anti-microbial, anti-fungal and anti-viral drugs.

A further preferred drug is one that is used to treat Dry Eye. An example of such a drug is Topical Cyclosporine A ("tcsA") 0.05% ophthalmic emulsion, sold in the USA under the name Restasis.

The uses and compositions described herein are useful in both human (medical) and animal (veterinary) applications. Preferred veterinary applications include the treatment of pets, such as cats and dogs, and other animals such as cattle, swine and horses.

The invention is further described by reference to the following non-limiting example.

Example - Study to evaluate the efficacy and tolerability of SPHP700 in the management of Dry Eye.

### Materials and Methods

SPHP700, a novel, single-dose, sterile ophthalmic solution for dry eye, was tested in this study. SPHP700 contains a natural polysaccharide of relatively high molecular weight extracted and filtered from linseed (Linum usitatissimum), combined with the polymer PVP in order to provide prolonged wetting and lubrication. The formulation of SPHP700 is given in Table 3, above.

The product's pH ranges between 7.2 - 7.6, while osmolality is between 270 and 300 mOsm/kg.

19 Patients diagnosed with dry eye condition were enrolled. 15 patients completed the study.

The endpoints evaluated were:
■ Schirmer's test
■ Tear Break Up Time (TBUT)
■ Redness
■ Symptomatology (dryness, blurred vision, irritation, sandy/gritty sensation, burning/stinging, light sensitivity, itching, foreign body sensation).

Each of these symptoms was scored using an ordinary 0 to 3 severity scale, where 0=none, 1=mild, 2=moderate, 3=severe, separately for right and left eye.

Each patient has to apply 1 eye drop of SPHP700 for each eye at least 3 times a day. Patients' visits were scheduled at 7 days (Visit 2) and 21 days (Visit 3) after the beginning of the treatment.

### Results

Of the 19 patients enrolled. 15 patients (79%) completed this pilot study, 4 males and 11 females aged between 36 and 83 years of age, (Mean 64.5 ±SD 11.5).

4 patients (21 %) withdrew from the study for the following reasons:
■ 1 Adverse Event was reported (Trigeminal neuralgia) but not related to SPHP700 as confirmed by the investigator.
■ 2 patients withdrew their consent to the study.
■ 1 patient was diagnosed with other unrelated eye complications and therefore switched to an antibacterial ophthalmic solution.

A particularly good improvement of TBUT was noted in 4 patients. The Schirmer's test showed inconsistent results, in that it did not always correspond to symptomatology improvements reported by the patients. However Schirmer's test has been cited in literature as not always reliable in dry eye diagnosis.

Overall, patients reported a marked improvement, whether partial or complete, of all clinical symptoms. In only one case and only for one symptom there was a worsening after 3 weeks, compared to baseline.

The percentage of patients who reported overall relief in symptoms, whether partial or complete was 93% at Visit 2. The percentage rose to 100% at Visit 3. In addition, patient # 17 and # 24 reported a relief lasting longer than 6 hours at Visit 3. Even patients who stated with a milder overall score (≤10) showed a good improvement.

Every patient had a normal intraocular pressure at the end of the study.

On balance, the patients reported very good results. These results were achieved by using the product up to 3 times per day (t.i.d.). This demonstrates the efficacy of a product containing linseed extract (and polyvinylpyrrolidone) in treating Dry Eye Syndrome.

## Claims

1. Use of a linseed extract in the manufacture of a medicament for the treatment of Dry Eye.

2. Use according to claim 1, wherein the medicament comprises polyvinylpyrrolidone.

3. Use according to clam 1 or claim 2, wherein the medicament comprises hyaluronic acid or a pharmaceutically acceptable salt thereof.

4. Use according to claim 2, wherein the medicament has the composition selected from:
a) 0.5% w/v Polyvinylpyrrolidone
0.65% w/v Sodium Chloride
0.5% w/v Linseed Dried Extract
0.034% w/v Sodium Phosphate
0.236% w/v Sodium Diphosphate Anhydrous
Remainder Distilled Water;
b) 0.5% w/v Polyvinylpyrrolidone
0.54% w/v Sodium Chloride
1% w/v Linseed Dried Extract
0.066% w/v Sodium Phosphate
0.471% w/v Sodium Diphosphate Anhydrous
Remainder Distilled Water;
c) 2% w/v Polyvinylpyrrolidone
0.54% w/v Sodium Chloride
1% w/v Linseed Dried Extract
0.066% w/v Sodium Phosphate
0.4715% w/v Sodium Diphosphate Anhydrous
Remainder Distilled Water.

5. A composition comprising linseed extract for use in the treatment of Dry Eye.

6. A composition according to claim 5, further comprising polyvinylpyrrolidone.

7. A composition according to claim 5 or 6, further comprising hyaluronic acid or a pharmaceutically acceptable salt thereof.

8. A composition according to any of claims 5 to 7, wherein the composition is as defined in claim 4.

## Patentansprüche

1. Verwendung eines Leinsamenextrakts bei der Herstellung eines Arzneimittels zur Behandlung des Trockenen Auges.

2. Verwendung nach Anspruch 1, wobei das Arzneimittel Polyvinylpyrrolidon umfasst.

3. Verwendung nach Anspruch 1 oder 2, wobei das Arzneimittel Hyaluronsäure oder ein pharmazeutisch verträgliches Salz davon umfasst.

4. Verwendung nach Anspruch 2, wobei das Arzneimittel eine Zusammensetzung aufweist, die ausgewählt ist aus:
a) 0,5 % Gew./Vol. Polyvinylpyrrolidon
0,65 % Gew./Vol. Natriumchlorid
0,5 % Gew./Vol. Leinsamen-Trockenextrakt
0,034 % Gew./Vol. Natriumphosphat
0,236 % Gew./Vol. Natriumdiphosphat, wasserfrei Rest destilliertes Wasser;
b) 0,5 % Gew./Vol. Polyvinylpyrrolidon
0,54 % Gew./Vol. Natriumchlorid
1 % Gew./Vol. Leinsamen-Trockenextrakt
0,066 % Gew./Vol. Natriumphosphat
0,471 % Gew./Vol. Natriumdiphosphat, wasserfrei Rest destilliertes Wasser; oder
c) 2 % Gew./Vol. Polyvinylpyrrolidon
0,54 % Gew./Vol. Natriumchlorid
1 % Gew./Vol. Leinsamen-Trockenextrakt
0,066 % Gew./Vol. Natriumphosphat
0,4715 % Gew./Vol. Natriumdiphosphat, wasserfrei Rest destilliertes Wasser.

5. Zusammensetzung, umfassend Leinsamenextrakt zur Verwendung bei der Behandlung des Trockenen Auges.

6. Zusammensetzung nach Anspruch 5, ferner umfassend Polyvinylpyrrolidon.

7. Zusammensetzung nach Anspruch 5 oder 6, ferner umfassend Hyaluronsäure oder ein pharmazeutisch verträgliches Salz davon.

8. Zusammensetzung nach einem der Ansprüche 5 bis 7, wobei die Zusammensetzung wie nach Anspruch 4 definiert ist.

## Revendications

1. Utilisation d'un extrait de graine de lin dans la fabrication d'un médicament pour le traitement du syndrome de l'oeil sec.

2. Utilisation selon la revendication 1, dans laquelle le médicament contient de la polyvinylpyrrolidone.

3. Utilisation selon la revendication 1 ou la revendication 2, dans laquelle le médicament contient de l'acide hyaluronique ou un sel pharmaceutiquement acceptable de celui-ci.

4. Utilisation selon la revendication 2, dans laquelle la composition du médicament est sélectionnée parmi :
a) 0,5% p/v de polyvinylpyrrolidone
0,65% p/v de chlorure de sodium
0,5% p/v d'extrait sec de graine de lin
0,034% p/v de phosphate de sodium
0,236% p/v de diphosphate de sodium anhydre
Reste constitué d'eau distillée ;
b) 0,5% p/v de polyvinylpyrrolidone
0,54% p/v de chlorure de sodium
1% p/v d'extrait sec de graine de lin
0,066% p/v de phosphate de sodium
0,0471% p/v de diphosphate de sodium anhydre
Reste constitué d'eau distillée ;
c) 2% p/v de polyvinylpyrrolidone
0,54% p/v de chlorure de sodium
1 % p/v d'extrait sec de graine de lin
0,066% p/v de phosphate de sodium
0,4715% p/v de phosphate de sodium anhydre
Reste constitué d'eau distillée

5. Composition contenant un extrait de graine lin pour utilisation dans le traitement du syndrome de l'oeil sec.

6. Composition selon la revendication 5, contenant en outre de la polyvinylpyrrolidone.

7. Composition selon la revendication 5 ou 6, contenant de l'acide hyarulonique ou un sel pharmaceutiquement acceptable de celui-ci.

8. Composition selon l'une quelconque des revendications 5 à 7, dans laquelle la composition est définie dans la revendication 4.
